# EUROPEAN PATENT APPLICATION

(11) **EP 4 736 846 A1**
(43) Date of publication of application: **06.05.2026**
(21) Application number: 25209403.2
(22) Date of filing: 17.10.2025
(51) Int. Cl.: A61K 9/20, A61K 31/496

(54) **TABLET COMPOSITION COMPRISING BREXPIPRAZOLE OR A SALT THEREOF**

(30) Priority: 01.11.2024 TR 2024014920
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); IPEK, Celaleddin, Istanbul (TR); ATAMAN, Seval, Istanbul (TR); GENCAL, Aynur, Istanbul (TR); SARP, Onder, Istanbul (TR); INAN, Fevzi, Istanbul (TR)
(74) Representative: Genç Ilhan, Oznur

(57) **Abstract**

The present invention relates to a tablet composition comprises brexpiprazole or a salt thereof and at least one binder and at least one glidant, wherein glidant is colloidal silicon dioxide. Further, the present invention also relates to a simple, rapid, cost effective, time-saving and industrially convenient process.

## Description

### Field of the Invention

The present invention relates to a tablet composition comprises brexpiprazole or a salt thereof and at least one binder and at least one glidant, wherein glidant is colloidal silicon dioxide. Further, the present invention also relates to a simple, rapid, cost effective, time-saving and industrially convenient process.

### Background of the Invention

Brexpiprazole acts as a partial agonist of the serotonin 5-HT1A receptor and the dopamine D2 and D3 receptors. Brexpiprazole has been described for use in the treatment of schizophrenia, depression and other central nervous system disorders, and for use in the prophylaxis and/or treatment of behavioral and psychological symptoms associated with neurodegenerative disease or impulsive symptoms associated with mental disease.

Brexpiprazole is a compound represented by the following Formula I, and its chemical name is 7-[4-[4-(1-benzothiophen-4-yl)piperazin-1-yl]butoxy]-1H-quinolin-2-one.

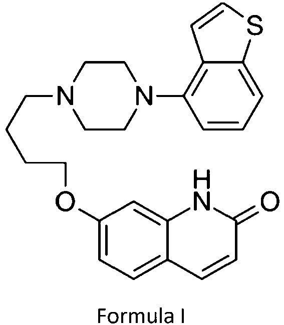

Brexpiprazole is an antidepressant and antipsychotic drug marketed under the brand Rexulti^{®} for the oral treatment of schizophrenia and as an adjunctive treatment to antidepressants in major depressive disorder. REXULTI tablets are intended for oral administration and available in 0.25 mg, 0.5 mg, 1 mg, 2 mg, 3 mg and 4 mg strengths. The product was approved in the U.S. in 2015 for the aforementioned indications and is currently in phase III trials for the treatment of agitation associated with Alzheimer's disease and the treatment of PTSD (post-traumatic stress disorder).

Brexpiprazole has practically insoluble in water. The major limitation of the drug is its low solubility. So, in a formulation comprising brexpiprazole, solubility is important.

WO2019121849 patent application relates to a pharmaceutical composition in the form of a tablet, comprising a) brexpiprazole as active ingredient and b) at least 5% by weight (wt%) pregelatinized starch (PGS). The invention relates to methods of preparing the tablet comprising dry granulation of a blend of the components to produce granules and compression of the granules to a tablet.

EP3545950A1 patent application relates to a pharmaceutical composition comprising a granulate comprising brexpiprazole, wherein the granulate is obtained by wet-granulation of a carrier using a granulation liquid that is a solution of brexpiprazole in a solvent system.

In prior art, low solubility of brexpiprazole has been approached in several ways. However, we have seen that studies carried out to correct the dissolution profile may cause some stability problems.

There is thus still a need for a film coated tablet comprising brexpiprazole or a salt thereof that provides a tablet having the desired stability, dissolution profile, hardness in another words the disadvantages seen in the active substance will be able to be overcome. The formulation has been developed by using standard techniques which is simple and cost-effective method.

### Detailed Description of the Invention

The main object of the present invention is to eliminate problems caused by active substance, brexpiprazole, and bringing additional advantages to the relevant prior art.

Another object of the present invention is to provide a tablet comprising brexpiprazole with content uniformity and hardness and having desired level of dissolution profile.

Another object of the present invention is to provide a tablet comprising brexpiprazole having improved high stability.

The present invention is based on the surprising finding that a particular tablet composition comprising brexpiprazole or a salt thereof can be provided by using colloidal silicon dioxide to promote disintegration.

Furthermore, the compositions of the present invention show high stability and physical integrity, e.g. during storage, handling, packaging and the like, without limiting the disintegration performance of the composition. The inclusion of colloidal silicon dioxide in a suitable amount is additionally advantageous because it results in a composition which, when compressed into a tablet, possesses enhanced mechanical properties. In particular, tablets formed from compositions according to the present invention possess a surprising combination of rapid disintegration with mechanical stability.

For a given level of hardness, the inclusion of colloidal silicon dioxide results in tablets having a faster disintegration rate.

Moreover, in this invention, we have seen that with the use of at least one binder, the desired content uniformity and dissolution profile is achieved.

According to the main embodiment of the present invention, a tablet composition comprises brexpiprazole or a salt thereof and at least one binder and at least one glidant, wherein glidant is colloidal silicon dioxide and the amount of colloidal silicon dioxide in the tablet is 0.05% to 3.0% by weight of the total composition.

Suitable binders are selected from the group comprising polyvinylpyrrolidone, carboxymethylcellulose sodium, sugars, agar, alginates, carbomers, cellulose acetate phthalate, chitosan, acacia mucilage, dextrates, dextrin, dextrose, ethylcellulose, glyceryl behenate, hydroxyethyl cellulose, hydroxyethylmethyl cellulose, hydroxypropyl methyl cellulose, hydroxypropyl starch, magnesium aluminum silicate, maltodextrin, methylcellulose, poloxamer, polycarbophil, polydextrose, polyethylene oxide, polymethacrylates, stearic acid, sucrose, polyoxyethylene-alkyl ethers, pullulan or mixtures thereof.

According to this embodiment of the present invention, the binder is hydroxypropyl methyl cellulose. Especially, binder is hydroxypropyl methyl cellulose (K4M XR).

According to this embodiment of the present invention, the amount of binder in the tablet is 0.1% to 5.0% by weight of the total composition. Preferably, it is 0.5% to 3.0% by weight of the total composition.

According to one embodiment of the present invention, the amount of brexpiprazole or a salt thereof is between 0.1% and 10.0% by weight of the total composition.

According to this embodiment of the present invention, the tablet further comprises at least one pharmaceutically acceptable excipient which is selected from the group comprising fillers, disintegrants, lubricants or mixtures thereof.

According to one embodiment of the present invention, fillers are selected from group comprising lactose monohydrate, microcrystalline cellulose, maize starch, mannitol, pregelatinized starch, ammonium alginate, calcium carbonate, anhydrous lactose, calcium phosphate, calcium phosphate dihydrate, neutral pellets, calcium sulfate, cellulose, ethylcellulose, fructose, glyceryl palmitostearate, magnesium carbonate, magnesium oxide, maltodextrin, maltose, medium chain triglycerides, polydextrose, polymethacrylates, sodium alginate, sodium chloride, sorbitol, starch, sucrose, sugar sphericals, talc, polysorbate 80, xylitol or mixtures thereof.

According to this embodiment of the present invention, filler is lactose monohydrate, microcrystalline cellulose, maize starch or mixtures thereof.

According to this embodiment of the present invention, the amount of filler in the tablet is 80.0% to 95.0% by weight of the total composition. Preferably, it is 80.0% to 90.0% by weight of the total composition.

According to this embodiment of the present invention, the amount of lactose monohydrate is 40.0% to 60.0% by weight of the total composition. Preferably, it is 45.0% to 55.0% by weight of the total composition.

According to this embodiment of the present invention, the amount of microcrystalline is 5.0% to 25.0% by weight of the total composition. Preferably, it is 8.0% to 20.0% by weight of the total composition.

According to this embodiment of the present invention, the amount of maize starch is 10.0% to 32.0% by weight of the total composition. Preferably, it is 15.0% to 28.0% by weight of the total composition.

Suitable disintegrants are selected from the group comprising sodium starch glycolate, crospovidone, cross-linked carboxymethyl cellulose (croscarmellose sodium), low-substituted hydroxypropyl cellulose, carboxymethyl cellulose, docusate sodium, guar gum, sodium alginate, alginic acid, magnesium aluminium silica, poloxamer, sodium glycine carbonate or mixtures thereof.

According to this embodiment of the present invention, the disintegrant is low-substituted hydroxypropyl cellulose. This helps to provide the desired dissolution profile.

According to this embodiment of the present invention, the amount of disintegrant in the tablet is 5.0% to 25.0% by weight, preferably 8.0% to 15.0% by weight of the total composition.

Suitable lubricants are selected from the group comprising sodium stearyl fumarate, magnesium stearate, calcium stearate, zinc stearate, talc, boric acid, hydrogenated vegetable oil, sodium chlorate, glycerol dibehenate, magnesium lauryl sulfate, sodium oleate, sodium acetate, sodium benzoate, polyethylene glycol, stearic acid, fatty acid, fumaric acid, glyceryl palmito sulphate, sodium lauryl sulphate or mixtures thereof.

According to this embodiment of the present invention, the lubricant is sodium stearyl fumarate. It improves the powder processing properties of tablet formulation. Also, it enhances powder flow by reducing the inter-particle friction.

According to this embodiment of the present invention, the amount of lubricant in the tablet is 0.7% to 3.0% by weight of the total composition.

According to this embodiment of the present invention, the tablet composition comprises;
▪ Brexpiprazole or a salt thereof
▪ At least one binder
▪ Colloidal silicon dioxide
▪ Sodium stearyl fumarate

According to this embodiment of the present invention, the tablet comprises;
▪ Brexpiprazole
▪ Lactose monohydrate
▪ Microcrystalline cellulose
▪ Maize starch
▪ Hydroxypropylcellulose
▪ Low-substituted hydroxypropylcellulose
▪ Colloidal silicon dioxide
▪ Sodium stearyl fumarate

According to one embodiment of the present invention the tablet comprises;
▪ Brexpiprazole
▪ Lactose monohydrate
▪ Microcrystalline cellulose
▪ Maize starch
▪ Hydroxypropyl methyl cellulose
▪ Low-substituted hydroxypropylcellulose
▪ Colloidal silicon dioxide
▪ Sodium stearyl fumarate

According to this embodiment of the present invention, the tablet is prepared wet granulation which is simple and cost-effective method. Solvent is used in the process.

Suitable solvents are selected from the group comprising water, ethanol, dichloromethane, 0.1N HCl, isopropyl alcohol, benzyl alcohol, propylene glycol, polyethylene glycol, glycerine, glycerine triacetate, diethylene glycol monoethyl ether, glycerol, propylene carbonate or mixtures thereof.

According to this embodiment of the present invention, the solvent during wet granulation is ethanol:water solution (70:30). The solvent provides to improve uniformity of content and to give shorter drying time for granules.

Furthermore, when only water was used in wet granulation, we observed that granules have stick problems. Ethanol:water solution (70:30) as solvent provides to improve the quality of granules in wet granulation and sticky problems were not observed.

### Example 1: Tablet

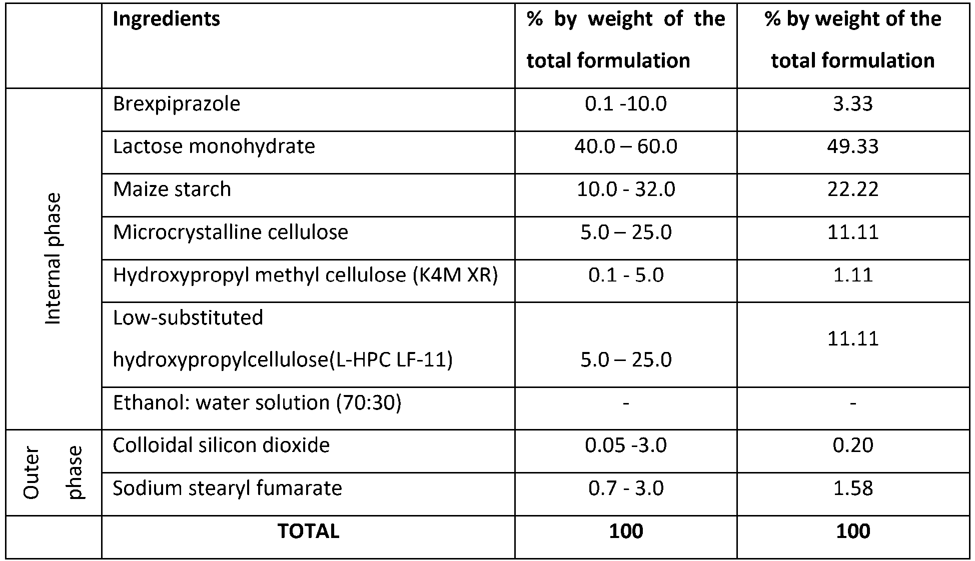

A process for example 1;
Internal phase;
   a) Sieving brexpiprazole, lactose monohydrate, maize starch and microcrystalline cellulose and low-substituted hydroxypropylcellulose into 0.6 mm and then mixing,
   b) Dissolving hydroxypropyl methyl cellulose into ethanol:water solution (70:30) and obtained a granulation solvent,
   c) Granulating the mixture at step (a) and the granulation solvent at step (b),
   d) Sieving the wet mixture into 4 mm,
   e) Drying the wet mixture until Loss on drying reaches under 2%,
   f) Sieving the dry mixture into 0.5 mm and then mixing,
Outer phase;
   g) Sieving Colloidal silicon dioxide into 0.6 mm and then adding into step (f) and mixing,
   h) Sieving Sodium stearyl fumarate into 0.6 mm and then adding into step (g) and then mixing,
   i) Compressing the total mixture into tablet form.

## Claims

1. A tablet composition comprising brexpiprazole or a salt thereof and at least one binder and at least one glidant, wherein glidant is colloidal silicon dioxide and the amount of colloidal silicon dioxide in the tablet is 0.05% to 3.0% by weight of the total composition.

2. The tablet composition according to claim 1, wherein binders are selected from the group comprising polyvinylpyrrolidone, carboxymethylcellulose sodium, sugars, agar, alginates, carbomers, cellulose acetate phthalate, chitosan, acacia mucilage, dextrates, dextrin, dextrose, ethylcellulose, glyceryl behenate, hydroxyethyl cellulose, hydroxyethylmethyl cellulose, hydroxypropyl methyl cellulose, hydroxypropyl starch, magnesium aluminum silicate, maltodextrin, methylcellulose, poloxamer, polycarbophil, polydextrose, polyethylene oxide, polymethacrylates, stearic acid, sucrose, , polyoxyethilene-alkyl ethers, pullulan or mixtures thereof.

3. The tablet composition according to claim 2, wherein, the binder is hydroxypropyl methyl cellulose.

4. The tablet composition according to claim 2 or 3, wherein the amount of binder in the tablet is 0.1% to 5.0% by weight of the total composition.

5. The tablet composition according to claim 1, wherein the amount of brexpiprazole or a salt thereof is between 0.1% and 10.0% by weight of the total composition.

6. The tablet composition according to any preceding claims, wherein further comprising at least one pharmaceutically acceptable excipient which is selected from the group comprising fillers, disintegrants, lubricants or mixtures thereof.

7. The tablet composition according to claim 6, wherein fillers are selected from group comprising lactose monohydrate, microcrystalline cellulose, maize starch, mannitol, pregelatinized starch, ammonium alginate, calcium carbonate, anhydrous lactose, calcium phosphate, calcium phosphate dehydrate, neutral pellets, calcium sulfate, cellulose, ethylcellulose, fructose, glyceryl palmitostearate, magnesium carbonate, magnesium oxide, maltodextrin, maltose, medium chain triglycerides, polydextrose, polymethacrylates, sodium alginate, sodium chloride, sorbitol, starch, sucrose, sugar sphericals, talc, polysorbate 80, xylitol or mixtures thereof.

8. The tablet composition according to claim 7, wherein filler is lactose monohydrate, microcrystalline cellulose, maize starch or mixtures thereof.

9. The tablet composition according to claim 6, wherein disintegrants are selected from the group comprising sodium starch glycolate, crospovidone, cross-linked carboxymethyl cellulose (croscarmellose sodium), low-substituted hydroxypropyl cellulose, carboxymethyl cellulose, docusate sodium, guar gum, sodium alginate, alginic acid, magnesium aluminium silica, poloxamer, sodium glycine carbonate or mixtures thereof.

10. The tablet composition according to claim 9, wherein the disintegrant is low-substituted hydroxypropyl cellulose.

11. The tablet composition according to claim 6, wherein lubricants are selected from the group comprising sodium stearyl fumarate, magnesium stearate, calcium stearate, glycerol dibehenate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmito sulphate, sodium lauryl sulphate or mixtures thereof.

12. The tablet composition according to claim 11, wherein the lubricant is sodium stearyl fumarate.

13. The tablet composition according to claim 12, wherein the composition comprising;
▪ Brexpiprazole or a salt thereof
▪ At least one binder
▪ Colloidal silicon dioxide
▪ Sodium stearyl fumarate

14. The tablet composition according to claim 6, wherein the tablet comprising;
▪ Brexpiprazole
▪ Lactose monohydrate
▪ Microcrystalline cellulose
▪ Maize starch
▪ Hydroxypropyl methyl cellulose
▪ Low-substituted hydroxypropylcellulose
▪ Colloidal silicon dioxide
▪ Sodium stearyl fumarate
